Europäisches Patentamt

European Patent Office

Office européen des brevets

(19)

(11) Publication number: **0 057 075**
**B1**

(12) **EUROPEAN PATENT SPECIFICATION**

(45) Date of publication of patent specification: **06.06.84**

(51) Int. Cl.³: **C 07 J 9/00**

(21) Application number: **82300240.7**

(22) Date of filing: **18.01.82**

(54) **A process for the isolation of beta-sitosterol from a steroid mixture.**

(30) Priority: **27.01.81 FI 810216**

(43) Date of publication of application:
**04.08.82 Bulletin 82/31**

(45) Publication of the grant of the patent:
**06.06.84 Bulletin 84/23**

(84) Designated Contracting States:
**AT BE CH DE FR GB IT LI NL SE**

(56) References cited:
**FR - A - 2 470 135**

**CHEMICAL ABSTRACTS, vol. 94, no. 11, March
16, 1981, abstract no. 79905w, page 353
COLUMBUS, OHIO (US)
CHEMICAL ABSTRACTS, vol. 94, no. 21, May
25, 1981, abstract no. 175373s, page 759
COLUMBUS, OHIO (US)**

(73) Proprietor: **Farmos-Yhtymä Oy
P.O. Box 425
SF-20101 Turku 10 (FI)**

(72) Inventor: **Koskenniska, Lasse Antero
Liistetie 4 A 2
SF-90650 Oulu (FI)**

(74) Representative: **Collier, Jeremy Austin Grey et al,
J.A.Kemp & Co. 14, South Square Gray's Inn
London WC1R 5EU (GB)**

## Description

This invention relates to the isolation of β-sitosterol from a steroid mixture, especially such a mixture separated from the neutral fraction of raw-soap. By using the process of this invention, it is possible to obtain β-sitosterol which contains at most 4%, and generally below 2%, α-sitosterol and below 7% campesterol. The amount of other by-products together is below 0.5%.

β-Sitosterol can be used as such in the pharmaceutical industry as a medicine and as a raw material for producing other steroids. It is however of great importance that the β-sitosterol shall fulfil certain quality demands. In particular it is important to obtain a β-sitosterol which contains the lowest possible amount of α-sitosterol, and certainly less than 4%. Natural sources of β-sitosterol, as for instance the sitosterol mixture separated from the neutral fraction of raw soap which originates from the sulphate cellulose process, may contain a substantial proportion, 20% or more, of α-sitosterol.

The separation of α- and β-sitosterol is therefore in practice an important task. For the solution of this problem the following methods have been proposed:

In United States Patent No. 2 573 265 a process is described for the separation of sitosterols, including β-sitosterol, in which the starting material is dissolved in a hydrocarbon or a halogenated hydrocarbon, after which β-sitosterol and isomeric sitosterols are crystallized with the aid of $HClO_4$ or $HPF_6$.

A method for separating α- and β-sitosterol chromatographically on aluminium oxide is described in the publication Sci. Res. (Dakka, Pak.) 1969 p. 162.

In Finnish Patent publication No. 57956 a process is described for the separation of β-sitosterol from mixtures that contain inter alia α-sitosterol. It is a characteristic of this process that the raw-material is treated with a strong inorganic or organic acid in an organic solvent. After the acid treatment the reaction product, which is obtained for instance by chilling the solution or by evaporating the solvent, is crystallized from a suitable organic solvent. If a steroid mixture, separated from the neutral fraction of raw soap, is used as raw material, a final product is obtained by this process, which contains about 90% β-sitosterol, about 6% campesterol and products derived from the decomposition or polymerisation of α-sitosterol and perhaps also products formed by splitting off water. If the conditions are chosen correctly, only very little or under 2% of the original α-sitosterol is left in the final product.

In Finnish Patent Publication No. 58333 a method is described for removing α-sitosterol from a product rich in β-sitosterol, in which the product rich in β-sitosterol is treated with a solvent mixture containing an aromatic and/or aliphatic hydrocarbon and esters, and possibly also small amounts of ketones, alcohols, organic acids and water. One disadvantage of this method is that the yield of β-sitosterol is quite poor, that is under 55%, and at the same time the α-sitosterol content in the final product is over 2%.

The process of this invention for the separation of β-sitosterol from a raw steroid mixture, which in addition to β-sitosterol may contain α-sitosterol and campesterol, tries to eliminate the above-mentioned drawbacks. By the process of this invention β-sitosterol can be separated from the steroid mixture in yields of over 90%, and the purified β-sitosterol obtained may contain 0—4.0% α-sitosterol and 6.0—7.0% campesterol.

According to the present invention β-sitosterol is isolated as a hydrate from a steroid mixture containing β-sitosterol by dissolving the said mixture in isopropanol or acetone, the weight ratio of steroid mixture to solvent being 1:8 to 1:13 and then adding water to the said solution in a volume ratio of water added to isopropanol of 1:5 to 1:20 or of water added to acetone of 1:20 to 1:100 so as to precipitate the said β-sitosterol as a hydrate. The steroid mixture is preferably dissolved in the acetone or isopropanol at elevated temperature. The crystallized steroid obtained contains 2—4% water as a hydrate, and this can only be removed by heating to 80—100°C.

Acetone and isopropanol are cheap solvents and they can be reused after distillation. The process of invention is economical and easy to perform because complicated solvent mixtures are not used, as are often required for other purification processes. The use of only one solvent is a noteworthy advantage when the process is performed on a technical scale. The solvents used, i.e. acetone and isopropanol, are also advantageous from the point of view of safety at work. Another advantage is that the final product does not contain in addition to β-sitosterol any other impurities than the above mentioned campesterol and α-sitosterol. The final product is thus suitable as such for pharmaceutical purposes.

The new process may preferably be operated as follows. The raw steroid mixture, containing β-sitosterol, is refluxed for 1/2—1 hour with isopropanol or acetone until the mixture has gone into solution. The weight ratio of the steroid mixture to the solvent is 1:8 to 1:13. Water is added to the solution so that β-sitosterol forms a hydrate which crystallizes upon cooling. When the solvent is isopropanol the volume ratio of water added to isopropanol is 1:5 to 1:20 while if the solvent is acetone the corresponding ratio is preferably 1:20 to 1:100. Most of the α-sitosterol and the neutral components occurring at low concentrations, which are harmful impurities, remain in the mother liquor. The crystallized fraction contains over 90% β-sitosterol, 6—7% campesterol and at

most 4% $\alpha$-sitosterol. The solvent from the mother liquor is easily recovered by distillation. Consequently, there are no problems in the refining process with the recovery and the circulation of the solvents. The concentration of campesterol has no influence on the success of the refining process, because campesterol also solidifies as a hydrate almost quantitatively.

The process of invention is described in detail in the following Examples. In Examples 1—5 a raw steroid mixture is used separated from the neutral fraction of raw soap and having the following composition:

| | |
|---|---|
| $\beta$-sitosterol | 76.2% |
| $\alpha$-sitosterol | 17.7% |
| Campesterol | 6.1% |

In Example 6 another raw steroid mixture, separated in the same way, is used as starting material having the composition:

| | |
|---|---|
| $\beta$-sitosterol | 87.9% |
| $\alpha$-sitosterol | 6.1% |
| Campesterol | 6.0% |

The following Examples illustrate the invention.

### Example 1

10 g of raw $\beta$-sitosterol, containing 76.2% $\beta$-sitosterol, 17.7% $\alpha$-sitosterol and 6.1% campesterol, were dissolved in 100 ml of hot acetone. To the solution was added 5 ml of water and the mixture was cooled to 10°C. The precipitate formed was filtered off and dried. The precipitate weighed 7.9 g and contained 91.5% $\beta$-sitosterol, 2% $\alpha$-sitosterol, and 6.5% campesterol.

### Example 2

10 g of raw $\beta$-sitosterol (as in Example 1) were dissolved in 100 ml of hot acetone. To the solution was added 1 ml of water and the mixture was cooled to 10°C. The precipitate formed was filtered off and dried. The precipitate weighed 8.2 g and contained 89.0% $\beta$-sitosterol, 4.0% $\alpha$-sitosterol, and 7% campesterol.

### Example 3

10 g of raw $\beta$-sitosterol (as in Example 1) were dissolved in 100 ml of hot isopropanol, 20 ml of water were added to the solution and the mixture was cooled to 10°C. The precipitate formed was filtered off and dried. The precipitate weighed 7.8 g and contained 91.9% $\beta$-sitosterol, 1.5% $\alpha$-sitosterol, and 6.6% campesterol.

### Example 4

10 g of raw $\beta$-sitosterol (as in Example 1) were dissolved in 80 ml of hot isopropanol. To the solution was added 10 ml of water and the mixture was cooled to 10°C. The precipitate formed was filtered off and dried. The precipitate weighed 7.7 g and contained 92.6% $\beta$-sitosterol, 0.9% $\alpha$-sitosterol and 6.5% campesterol.

### Example 5

10 g of raw $\beta$-sitosterol (as in Example 1) were dissolved in 100 ml of hot isopropanol. To the solution was added 1 ml of water. The solution was cooled to 10°C and the precipitate formed was filtered off and dried. The precipitate weighed 7.6 g and contained 93.9% $\beta$-sitosterol, 0.5% $\alpha$-sitosterol and 6.6% campesterol.

### Example 6

10 g of raw $\beta$-sitosterol, which contained 87.9% $\beta$-sitosterol, 6.1% $\alpha$-sitosterol and 6.0% campesterol, were dissolved in 100 ml of hot isopropanol. To the solution was added 10 ml of water and the mixture was cooled to 10°C. The precipitate formed was filtered off and dried. The precipitate weighed 8.74 g and contained 93.4% $\beta$-sitosterol, 0.0% $\alpha$-sitosterol and 6.6% campesterol.

## Claims

1. Process for the isolation of $\beta$-sitosterol as a hydrate from a steroid mixture containing $\beta$-sitosterol which comprises dissolving the said mixture in isopropanol or acetone, the weight ratio of steroid mixture to solvent being 1:8 to 1:13 and then adding water to the said solution in a volume ratio of water added to isopropanol of 1:5 to 1:20 or of water added to acetone of 1:20 to 1:100 so as to precipitate the said $\beta$-sitosterol as a hydrate.

2. Process according to claim 1, in which the said mixture is dissolved in the said solvent at elevated temperature.

3. Process according to claim 1 or 2, in which the said steroid mixture contains $\beta$-sitosterol, $\alpha$-sitosterol and campesterol and the product obtained contains $\beta$-sitosterol, at most 4% by weight of $\alpha$-sitosterol, at most 7% by weight of campesterol, and at most 0.5% by weight of other impurities.

## Patentansprüche

1. Verfahren für die Isolierung von $\beta$-Sitosterol als Hydrat aus einer $\beta$-sitosterol-enthaltenden Steroidmischung, das di Auflösung der Mischung in Isopropanol oder Azeton, wobei das Gewichtsverhältnis der Steroidmischung zum Lösungsmittel 1:8 bis 1:13 ist, und dann Zufügen von Wasser zu der Lösung in einem Volumenverhältnis von dem Isopropanol zugefügten Wasser von 1:5 bis 1:20 oder von dem Azeton zugefügten Wasser von 1:20 bis 1:100, so daß sich das $\beta$-Sitosterol als Hydrat niederschlägt, enthält.

2. Verfahren nach Anspruch 1, bei dem die Mischung in dem Lösungsmittel bei erhöhter Temperatur aufgelöst wird.

3. Verfahren nach Anspruch 1 oder 2, bei dem die Steroidmischung β-Sitosterol, α-Sitosterol und Campesterol enthält und das erhaltene Produkt β-Sitosterol, höchstens 4 Gew.-% α-Sitosterol, höchstens 7 Gew.-% Campesterol und höchstens 0,5 Gew.-% andere Verunreinigungen enthält.

**Revendications**

1. Procédé pour isoler le β-sitostérol, sous forme d'un hydrate, d'un mélange de stéroïdes contenant du β-sitostérol, qui consiste à dissoudre ledit mélange dans l'isopropanol ou l'acétone, le rapport pondéral du mélange de stéroïdes au solvant étant de 1:8 à 1:13, puis à ajouter de l'eau à ladite solution dans un rapport en volume de l'eau ajoutée à l'isopropanol de 1:5 à 1:20, ou de l'eau ajoutée à l'acétone de 1:20 à 1:100, afin de précipiter ledit β-sitostérol sous forme d'un hydrate.

2. Procédé selon la revendication 1, dans lequel ledit mélange est dissous dans ledit solvant à température élevée.

3. Procédé selon les revendications 1 ou 2, dans lequel ledit mélange de stéroïdes contient du β-sitostérol de l'α-sitostérol, et du campestérol et le produit obtenu contient du β-sitostérol, au maximum 4% en poids d'α-sitostérol, au maximum 7% en poids de campostérol, et au maximum 0,5% en poids d'autres impuretés.